# EUROPEAN PATENT SPECIFICATION

(11) **EP 2 620 153 B1**
(45) Date of publication and mention of the grant of the patent: **18.04.2018**
(21) Application number: 11826361.5
(22) Date of filing: 08.07.2011
(51) Int. Cl.: A61K 31/565, A61K 9/08, A61K 9/14, A61K 9/19, A61K 47/40, A61P 9/00, A61P 25/00

(54) **5 ALPHA-ANDROSTANE (ALKYL)-3 BETA,5,6 BETA-TRIOL INJECTION AND PREPARATION METHOD THEREFOR**
5-ALPHA-ANDROSTAN-(ALKYL-)3-BETA-5,6 BETA-TRIOL-INJEKTION UND HERSTELLUNGSVERFAHREN DAFÜR
INJECTION DE 5 ALPHA-ANDROSTANE-(ALKYL)-3 BETA,5,6 BETA-TRIOL ET SON PROCÉDÉ DE PRÉPARATION

(30) Priority: 21.09.2010 CN 201010292234
(43) Date of publication of application: 31.07.2013
(73) Proprietor: Guangzhou Cellprotek Pharmaceutical Co., Ltd., Guangzhou 510663 (CN)
(72) Inventor: YAN, Guangmei, Guangzhou Guangdong 510080 (CN); HU, Haiyan, Guangzhou Guangdong 510080 (CN); ZHANG, Jingxia, Guangzhou Guangdong 510080 (CN); QIU, Pengxin, Guangzhou Guangdong 510080 (CN); LI, Ling, Guangzhou Guangdong 510080 (CN); TIAN, Ning, Guangzhou Guangdong 510080 (CN)
(74) Representative: Petraz, Gilberto Luigi
(86) International application number: PCT/CN2011/076968
(87) International publication number: WO 2012/037834

(56) References cited:
- WO-A1-97/17992
- CN-A- 1 201 397
- CN-A- 1 706 501
- CHEN JINGBO ET AL: "Hemisuccination of Hydroxysterols", HECHENG HUAXUE / CHINESE JOURNAL OF SYNTHETIC CHEMISTRY, CHENGDU YUJI HUAXUESUO, CHINA, vol. 8, no. 5, 8 May 2000 (2000-05-08), pages 466-468, XP008169327, ISSN: 1005-1511
- CHEN JINGBO ET AL.: 'Hemisuccination of Hydroxysterols' HECHEG HUAXUE vol. 8, no. 5, 2000, pages 466 - 468, XP008169327

## Description

### FIELD OF THE INVENTION

The present invention is in the field of pharmaceutics and relates to 5α-androstane -3β,5,6β-triol injection and its preparation method.

### BACKGROUND OF THE INVENTION

5α-androstane-3β,5,6β-triol (hereinafter YC-6) is a newly-found neuronprotective compound. Currently, Acute Ischemic Stroke (AIS) is treated mainly by thrombolytic or neuronprotective therapy. Neuronprotective agents can reduce cerebral infarction area and avoid hemorrhage complication that may occur during thrombolytic or anticoagulant therapy. Moreover, it can be used even without any aetiological diagnosis and make early treatment possible. Neuronprotective agents have therefore attracted increasing attention in AIS research.

However, no neuronprotective agent has been proven to be safe and effective so far. A lot of compounds with potential value for clinical application are under clinical trials, including calcium channel blockers (CCB), calcium channel modulators, glutamate release inhibitors, γ-aminobutyric acid (GABA) receptor agonists, free radical scavengers, anti-intercellular adhesion molecule antibodies, and so on.

Among a large number of compounds, neuroactive steroids are increasingly attractive due to their extensive effect of neuron protection. In particular, the effect of compound YC-6, as a newly-found neuroprotective chemical entity, is not limited to neuron protection. It is effective against not only cerebral ischemia but also spinal cord ischemia at daily dose of 50-100 mg.

YC-6 is insoluble in water. Although its solubility is increased in conventional non-aqueous solvents or mixtures thereof, these solvents cause irritation, and the precipitation of YC-6 might occur when diluted by water. The efficacy and safety of YC-6 injection are therefore adversely affected and its use thereof is limited.

A known hemisuccination of Hydroxysterols is described in the following document: CHEN JINGBO ET AL: "Hemisuccination of Hydroxysterols", HECHENG HUAXUE / CHINESE JOURNAL OF SYNTHETIC CHEMISTRY, CHENGDU YUJI HUAXUESUO, CHINA, vol. 8, no. 5, 8 May 2000 (2000-05-08), pages 466-468, XP008169327, ISSN: 1005-1511.

Document WO 97/17992 A1 describes a known formulation for administration of steroid compounds.

### SUMMARY OF THE INVENTION

To overcome the deficiencies set forth above, YC-6 injections and their preparation methods are provided by the present invention. The present invention uses hydroxypropyl-β-cyclodextrin as a solubilizing agent to prepare YC-6 injections. The irritation caused by non-aqueous solvents is successfully cut down while the solubility of YC-6 is increased.

To achieve this, YC-6 injections are provided in liquid or solid form. The injections have at least one soluble excipient including hydroxypropyl-β-cyclodextrin. The at least one soluble excipient can also comprise an isotonic adjusting agent or a freeze drying filler.

Preferably, YC-6 is present at a part by weight ratio of 1∼20:40∼500 to hydroxypropyl-β-cyclodextrin.

The injections can also be prepared by the following components (by weight): 1∼20 parts of YC-6, 40∼500 parts of hydroxypropyl-β-cyclodextrin, 1∼100 parts of an isotonic adjusting agent, 0∼200 parts of a freeze drying filler, and 0∼2000 parts of a solvent.

The isotonic adjusting agent is selected from the group consisting of sodium chloride, glucose, mannitol, lactose, xylitol, sorbitol, maltitol and mixtures thereof.

The freeze-drying filler is selected from the group consisting of sodium chloride, glucose, mannitol, lactose, xylitol, sorbitol, maltitol and mixtures thereof.

If a liquid injection is to be prepared, the solvent is selected from the group consisting of propanediol, ethanol, polyethylene glycol 400, polyethylene glycol 200, glycerol, water and mixtures thereof.

The injection of the present invention can be prepared by a method comprising steps of dissolving hydroxypropyl-β-cyclodextrin, YC-6 and at least one soluble excipient in water for injection in sequence to obtain a raw solution; and subjecting the raw solution to decolorization, filtration and sterilization in sequence to obtain the injection of the present invention.

Freeze-drying powder is prepared by filling a filtrate produced by the filtration step in the above into an ampoule and undergoing freeze drying.

Sterile powder is prepared by spray drying a filtrate produced by the filtration step in the above followed by packaging.

The decolorization can be performed by using 0.1∼ 0.3% activated carbon and the sterilization can be performed at 115°C for 30 min or at 121°C for 15 min.

It will be appreciated that the YC-6 can also be formulated into YC-6-loaded infusions, through mixing YC-6 injection with conventional drug-free infusions such as glucose infusion, sodium chloride infusion or glucose and sodium chloride infusion.

The present invention is advantageous over conventional techniques. Uses of hydroxypropyl-β-cyclodextrin or non-aqueous solvents/mixed solvents increase the solubility of YC-6, so that YC-6 can be prepared into injection solution, sterile powder, freeze drying powder, YC-6-loaded glucose infusion, sodium chloride infusion, or glucose and sodium chloride infusion, which makes possible for YC-6 to be administrated by intravenous injection in case of urgency. In addition, YC-6 has sufficient solubility and efficacy without any irritation when formulated into the present injection. The preparation process is also simple and widely available.

### DETAILED DESCRIPTION OF THE INVENTION

### Example 1 Preparation of 200 ampoules of YC-6 injection (specification 5ml: 50mg)

Formula:

| | |
|---|---|
| YC-6 | 10 g |
| 2-hydroxypropyl-β-cyclodextrin | 200 g |
| Sodium chloride | 1.25 g |
| Water for injection, added to | 1000 ml |

Preparation: Dissolve 2-hydroxypropyl-β-cyclodextrin in 80% of the fresh water for injection and add YC-6, followed by stirring at room temperature for 10∼20 min to make YC-6 completely dissolved. Add sodium chloride and make it dissolved by stirring, then add water for injection to 1000 ml. Add 0.1% activated charcoal into the above solution, stir for 15 min at 60 °C and then cool naturally the solution to room temperature. A 0.22µm microporous membrane filter is used to filtrate. The filtrate is collected and filled to prepare 5 ml injection, and subject to sterilization at 121°C for 15 min.

### Example 2 Preparation of 200 ampoules of YC-6 injection (specification 10ml: 80mg)

Formula:

| | |
|---|---|
| YC-6 | 16 g |
| 3-hydroxypropyl-β-cyclodextrin | 400 g |
| Glucose | 13.9 g |
| Water for injection, added to | 2000 ml |

Preparation: Dissolve 3-hydroxypropyl-β-cyclodextrin in 80% of the fresh water for injection and add YC-6, followed by stirring at room temperature for 10∼20 min to make YC-6 completely dissolved. Add glucose and make it dissolved by stirring, then add water for injection to 2000 ml. Add 0.1% activated charcoal into the above solution, and stir for 15 min at 60 °C and then cool naturally the solution to room temperature. A 0.22µm microporous membrane filter is used to filtrate. The filtrate is collected and filled to prepare 10 ml injection, and subject to sterilization at 121°C for 15 min.

### Example 3 Preparation of 200 ampoules of YC-6 injection (specification 5 ml: 100 mg)

Formula:

| | |
|---|---|
| YC-6 | 20 g |
| 2-hydroxypropyl-β-cyclodextrin | 400 g |
| Water for injection, added to | 1000 ml |

Preparation: Dissolve 2-hydroxypropyl-β-cyclodextrin in 80% of the fresh water for injection and add YC-6, followed by stirring at room temperature for 10∼20 min to make YC-6 completely dissolved. Add water for injection to 1000 ml. Add 0.1% activated charcoal into the above solution, and stir for 15 min at 60 °C and then cool naturally the solution to room temperature. A 0.22µm microporous membrane filter is used to filtrate. The filtrate is collected and filled to prepare 5 ml injection, and subject to sterilization at 115°C for 30 min.

### Example 4 Preparation of 200 ampoules of YC-6 sterile powder (specification 80 mg/bottle)

Formula:

| | |
|---|---|
| YC-6 | 16 g |
| 2-hydroxypropyl-β-cyclodextrin | 400 g |
| Sodium chloride | 2.5 g |
| Packaged into | 200 ampoules |

Preparation: Dissolve 2-hydroxypropyl-β-cyclodextrin in 80% of the fresh water for injection and add YC-6, followed by stirring at room temperature for 10∼20 min to make YC-6 completely dissolved. Add sodium chloride and make it dissolved by stirring, and add water for injection to 2000 ml. Add 0.1% activated charcoal into the above solution, and stir for 15 min at 60 °C and then cool naturally the solution to room temperature. A 0.22µm microporous membrane filter is used to filtrate. The filtrate is subject to spray drying and then packaged into 200 ampoules.

### Example 5 Preparation of 200 ampoules of YC-6 freeze drying powder (specification 5 ml: 60 mg)

Formula:

| | |
|---|---|
| YC-6 | 12 g |
| 3-hydroxypropyl-β-cyclodextrin | 200 g |
| Glucose | 7 g |
| Water for injection, added to | 1000 ml |

Preparation: Dissolve 3-hydroxypropyl-β-cyclodextrin in 80% of the fresh water for injection and add YC-6, followed by stirring at room temperature for 10∼20 min to make YC-6 completely dissolved. Add glucose and make it dissolved by stirring, and add water for injection to 1000 ml. Add 0.1% activated charcoal into the above solution, and stir for 15 min at 60 °C and then cool naturally the solution to room temperature. A 0.22µm microporous membrane filter is used to filtrate. The filtrate is packaged into 5 ml ampoules and then subject to freeze drying.

### Example 6 Compatible Stability of YC-6 injection and conventional infusions

Two ampoules of YC-6 injection (5 ml X 2) of Example 1 are added to conventional infusions to evaluate the compatible stability of YC-6 therein in 8 hours. Evaluating items of compatible stability include color, clarity, pH and YC-6 content. Results are shown in the following tables.

**Table 1. Compatibility tests of YC-6 injection and conventional infusions**

| ID | Compatibility tests (25-30°C) |
|---|---|
| A | YC-6 injection 5ml X 2+5% glucose injection 250 ml |
| B | YC-6 injection 5ml X 2+0.9% sodium chloride injection 250 ml |
| C | YC-6 injection 5ml X 2+glucose and NaCl injection 250 ml |
| D | YC-6 injection 5ml X 2+compound NaCl injection 500 ml |
| E | YC-6 injection 5ml X 2+5% sodium bicarbonate injection 250 ml |

**Table 2. Changes in color and clarity of conventional infusions**

| | | After adding (h) | | | |
|---|---|---|---|---|---|
| ID | Prior to adding | 0 | 2 | 4 | 8 |
| A | Colorless, clear | Colorless, clear | Colorless, clear | Colorless, clear | Colorless, clear |
| B | Colorless, clear | Colorless, clear | Colorless, clear | Colorless, clear | Colorless, clear |
| C | Colorless, clear | Colorless, clear | Colorless, clear | Colorless, clear | Colorless, clear |
| D | Colorless, clear | Colorless, clear | Colorless, clear | Colorless, clear | Colorless, clear |
| E | Colorless, clear | Colorless, clear | Colorless, clear | Colorless, clear | Colorless, clear |

**Table 3. pH changes of conventional infusions**

| | | After adding (h) | | | |
|---|---|---|---|---|---|
| ID | Prior to adding | 0 | 2 | 4 | 8 |
| A | 4.05 | 4.06 | 4.05 | 4.02 | 4.08 |
| B | 5.60 | 5.62 | 5.58 | 5.46 | 5.58 |
| C | 4.02 | 4.04 | 4.03 | 4.00 | 4.04 |
| D | 5.64 | 5.62 | 5.60 | 5.59 | 5.59 |
| E | 7.99 | 7.94 | 7.90 | 8.04 | 8.00 |

**Table 4. Changes of YC-6 concentration in conventional infusions**

| ID | 0h | 2h | 8h | 24h |
|---|---|---|---|---|
| A | 375.4 | 364.7 | 367.7 | 363.4 |
| B | 379.2 | 373.5 | 380.4 | 386.1 |
| C | 385.6 | 387.6 | 383.4 | 384.5 |
| D | 382.0 | 383.7 | 387.2 | 380.8 |
| E | 386.7 | 375.1 | 381.3 | 376.5 |

### Example 7 Preliminary Evaluation on the Security of YC-6

Kunming mice are caged by weight and randomly divided into 5 groups. Each group has 10 mice, with half males and half females. The YC-6 injection prepared in Example 3 (20 mg/ml) is *i.v.* administrated via tail vein at different doses. All mice are sacrificed after one-week observation. The toxic reaction and number of death of the animals are recorded every day. *LD₅₀* and 95% confidence are calculated. *LD₅₀* of YC-6 is more than 400 ± 121 mg/kg.

Blood cells are prepared according to conventional methods with fresh blood obtained from New Zealand rabbits. The blood cells are diluted with saline to a 2% suspension. The YC-6 injection prepared in Example 1 is then added to the 2% suspension and incubated at 37°C for 3 hours. Hemolysis rate is determined by using colorimetric method. Hemolysis rate of YC-6 injection is less than 1%.

Albino guinea pigs are subject to anaphylactic test according to conventional procedures. No anaphylactic reaction is observed after intravenous administration of the YC-6 injection prepared in Example 1.

New Zealand rabbits are used to perform vascular stimulation test of intravenous administration of the YC-6 injection prepared in Example 1. The results show that tissue changes of ear edge vein are similar between treatment group and control group. Each rabbit has integral vascular wall of ear edge vein and normal vein structure. No pathological change such as endothelial cells damage or surrounding tissue edema is observed.

## Claims

1. A 5α-androstane-3β,5,6β-triol injection, including a liquid injection having a solvent or a solid injection, comprises at least one soluble excipient, wherein the at least one soluble excipient includes hydroxypropyl-β-cyclodextrin.

2. The injection of claim 1, wherein the 5α-androstane-3β,5,6β-triol is present at a part by weight ratio of 1∼20: 40∼500 to hydroxypropyl-β-cyclodextrin.

3. The injection of claim 1 or 2, wherein the soluble excipient further comprises an isotonic adjusting agent and/or a freeze drying filler.

4. The injection of claim 3, wherein the isotonic adjusting agent is selected from the group consisting of sodium chloride, glucose, mannitol, lactose, xylitol, sorbitol, maltitol and mixtures thereof.

5. The injection of claim 3, wherein the freeze-drying filler is selected from the group consisting of sodium chloride, glucose, mannitol, lactose, xylitol, sorbitol, maltitol and mixtures thereof.

6. The injection of claim 1 or 2, wherein the solvent of the liquid injection is selected from the group consisting of propanediol, ethanol, polyethylene glycol 400, polyethylene glycol 200, glycerol, water and mixtures thereof.

7. The injection of claim 3, wherein the injection is consisted of (by weight): 1∼20 parts of 5α-androstane-3β,5,6β-triol, 40∼500 parts of hydroxypropyl-β-cyclodextrin, 1∼100 parts of an isotonic adjusting agent, 0∼200 parts of a freeze drying filler, and 0∼2000 parts of a solvent.

8. A method for preparing the injection of claim 1, comprising steps of
(a) dissolving hydroxypropyl-β-cyclodextrin, 5α-androstane-3β, 5,6β-triol and addtional soluble excipients in water for injection in sequence to obtain a raw injection solution, and
(b1) subjecting the raw injection solution to decolorization, depyrogenation, filtration and sterilization to obtain the injection, or
(b2) subjecting the raw injection solution to decolorization, depyrogenation, filtration and filling a filtrate produced by the filtration into an ampoule, followed by freeze drying to obtain the freeze drying powder, or
(b3) subjecting the raw injection solution to decolorization, depyrogenation, filtration and spray drying a filtrate produced by the filtration, followed by packaging.

9. The method of claim 8, wherein the decolorization is achieved by using activated charcoal at an amount of 0.05∼0.3 wt% of the injection.

10. The method of claim 8 or 9, wherein the sterilization is performed at 115°C for 30 min or at 121°C for 15 min.

## Patentansprüche

1. 5-α-Androstan-3β,5,6β-Triol-Injektion, umfassend eine flüssige Injektion, die ein Lösungsmittel umfasst, oder eine feste Injektion, die zumindest einen löslichen Hilfsstoff umfasst, worin der zumindest eine lösliche Hilfsstoff Hydroxypropyl-ß-Cyclodextrin umfasst.

2. Injektion nach Anspruch 1, worin das 5-α-Androstan-3β,5,6β-Triol in einem Gewichtsanteilverhältnis von 1∼20 : 40∼500 zum Hydroxypropyl-β-Cyclodextrin vorliegt.

3. Injektion nach Anspruch 1 oder 2, worin der lösliche Hilfsstoff ferner ein isotonisches Einstellmittel und/oder einen Gefriertrocknungsfüllstoff umfasst.

4. Injektion nach Anspruch 3, worin das isotonische Einstellmittel aus der Gruppe ausgewählt wird, die aus Natriumchlorid, Glukose, Mannit, Laktose, Xylit, Sorbit, Maltit und Mischungen davon besteht.

5. Injektion nach Anspruch 3, worin der Gefriertrocknungsfüllstoff aus der Gruppe ausgewählt wird, die aus Natriumchlorid, Glukose, Mannit, Laktose, Xylit, Sorbit, Maltit und Mischungen davon besteht.

6. Injektion nach Anspruch 1 oder 2, worin das Lösungsmittel der flüssigen Injektion aus der Gruppe ausgewählt wird, die aus Propandiol, Ethanol, Polyethylenglykol 400, Polyethylenglykol 200, Glyzerin, Wasser und Mischungen davon besteht.

7. Injektion nach Anspruch 3, worin die Injektion (gewichtsmäßig) aus Folgendem besteht: 1∼20 Teilen 5-α-Androstan-3β,5,6β-Triol, 40∼500 Teilen Hydroxypropyl-β-Cyclodextrin, 1∼100 Teilen eines isotonischen Einstellmittels, 0∼200 Teilen eines Gefriertrocknungsfüllstoffs und 0∼2000 Teilen eines Lösungsmittels.

8. Verfahren zur Herstellung der Injektion nach Anspruch 1, umfassend die Schritte des
(a) Auflösens von Hydroxypropyl-β-Cyclodextrin, 5-α-Androstan-3β, 5,6β-Triol und zusätzlichen löslichen Hilfsstoffen in Wasser zur Injektion in Reihenfolge, um eine rohe Injektionslösung zu erhalten, und
(b1) Unterziehens der rohen Injektionslösung einer Entfärbung, Entpyrogenisierung, Filtrierung und Sterilisierung, um die Injektion zu erhalten, oder
(b2) Unterziehens der rohen Injektionslösung einer Entfärbung, Entpyrogenisierung, Filtrierung und Füllen eines Filtrats, das durch die Filtrierung erzeugt wurde, in eine Ampulle, gefolgt von Gefriertrocknung, um das Gefriertrocknungspulver zu erhalten, oder
(b3) Unterziehens der rohen Injektionslösung einer Entfärbung, Entpyrogenisierung, Filtrierung und Sprühtrocknung eines Filtrats, das durch die Filtrierung erzeugt wurde, gefolgt von Verpackung.

9. Verfahren nach Anspruch 8, worin die Entfärbung durch Verwendung von Aktivkohle in einer Menge von 0,05∼0,3 Gew.-% der Injektion erhalten wird.

10. Verfahren nach Anspruch 8 oder 9, worin die Sterilisierung bei 115°C 30 Minuten lang oder bei 121°C 15 Minuten lang durchgeführt wird.

## Revendications

1. Une injection de 5α-androstane-3β,5,6β-triol, incluant une injection liquide ayant un solvant ou une injection solide, comprenant au moins un excipient soluble, dans laquelle le ou les excipient(s) soluble(s) inclu(en)t de l'hydroxypropyl-β-cyclodextrine.

2. L'injection selon la revendication 1, dans laquelle le 5α-androstane-3β,5,6β-triol est présent suivant un rapport massique de 1 à 20 : 40 à 500 par rapport à l'hydroxypropyl-β-cyclodextrine.

3. L'injection selon la revendication 1 ou 2, dans laquelle l'excipient soluble comprend en outre un agent d'ajustement isotonique et/ou un adjuvant de lyophilisation.

4. L'injection selon la revendication 3, dans laquelle l'agent d'ajustement isotonique est choisi dans le groupe constitué par le chlorure de sodium, le glucose, le mannitol, le lactose, le xylitol, le sorbitol, le maltitol et leurs mélanges.

5. L'injection selon la revendication 3, dans laquelle la charge de séchage par refroidissement est choisie dans le groupe constitué par le chlorure de sodium, le glucose, le mannitol, le lactose, le xylitol, le sorbitol, le maltitol et leurs mélanges.

6. L'injection selon la revendication 1 ou 2, dans laquelle le solvant de l'injection liquide est choisi dans le groupe constitué par le propane-diol, l'éthanol, le polyéthylène glycol 400, le polyéthylène glycol 200, le glycérol, l'eau et leurs mélanges.

7. L'injection selon la revendication 3, dans laquelle l'injection est constituée de (en poids) : 1 à 20 parties de 5α-androstane-3β,5,6β-triol, 40 à 500 parties d'hydroxypropyl-β-cyclodextrine, 1 à 100 parties d'agent d'ajustement isotonique, 0 à 200 parties d'un adjuvant de lyophilisation et 0 à 2000 parties d'un solvant.

8. Un procédé de préparation de l'injection selon la revendication 1, comprenant les étapes consistant à :
(a) dissoudre l'hydroxypropyl-β-cyclodextrine, le 5α-androstane-3β, le 5,6β-triol et les excipients solubles additionnels dans de l'eau pour une injection subséquente afin d'obtenir une solution d'injection brute, et
(bl) soumettre la solution d'injection brute à une décoloration, une dépyrogénation, une filtration et une stérilisation afin d'obtenir l'injection, ou
(b2) soumettre la solution d'injection brute à une décoloration, une dépyrogénation, une filtration et charger le filtrat obtenu par la filtration dans une ampoule, puis lyophiliser pour obtenir la poudre de lyophilisation, ou
(b3) soumettre la solution d'injection brute à une décoloration, une dépyrogénation, une filtration et un séchage par atomisation du filtrat obtenu par la filtration, puis effectuer un conditionnement.

9. Le procédé selon la revendication 8, dans lequel la décoloration est réalisée en utilisation un charbon actif à raison de 0,05 à 0,3% en poids par rapport à l'injection.

10. Le procédé selon la revendication 8 ou 9, dans lequel la stérilisation est effectuée à 115°C pendant 30 minutes ou à 121°C pendant 15 minutes.
